(19) Europäisches Patentamt
European Patent Office
Office européen des brevets

(11) EP 1 742 874 B1

(12) **EUROPEAN PATENT SPECIFICATION**

(45) Date of publication and mention of the grant of the patent:
**15.08.2012 Bulletin 2012/33**

(21) Application number: **04750375.0**

(22) Date of filing: **20.04.2004**

(51) Int Cl.:
***C01B 39/30*** *(2006.01)* ***C01B 39/02*** *(2006.01)*

(86) International application number:
**PCT/US2004/012154**

(87) International publication number:
**WO 2005/113438 (01.12.2005 Gazette 2005/48)**

(54) **UZM-16: A CRYSTALLINE ALUMINOSILICATE ZEOLITIC MATERIAL**

ZEOLITHMATERIAL AUF BASIS VON KRISTALLINEM ALUMINOSILICAT: UZM-16

UZM-16: UN MATÉRIAU ZÉOLITIQUE D'ALUMINOSILICATE CRISTALLIN

(84) Designated Contracting States:
**DE FR IT**

(43) Date of publication of application:
**17.01.2007 Bulletin 2007/03**

(73) Proprietor: **UOP LLC**
**Des Plaines, IL 60017-5017 (US)**

(72) Inventors:
- **MOSCOSO, Jaime G., UOP LLC Des Plaines, IL 60016 (US)**
- **SINKLER, Wharton, UOP LLC Des Plaines, IL 60016 (US)**
- **LEWIS, Gregory J., UOP LLC Des Plaines, IL 60016 (US)**
- **JAN, Deng Yang, UOP LLC Des Plaines, IL 60016 (US)**
- **KOSTER, Susan C., UOP LLC Des Plaines, IL 60016 (US)**

(74) Representative: **Hayes, Adrian Chetwynd et al**
**Boult Wade Tennant**
**Verulam Gardens**
**70 Gray's Inn Road**
**London WC1X 8BT (GB)**

(56) References cited:

| | |
|---|---|
| **GB-A- 1 589 856** | **US-A- 2 950 952** |
| **US-A- 3 699 139** | **US-A- 4 687 653** |
| **US-A- 4 834 961** | **US-A- 4 931 266** |
| **US-A- 5 133 951** | **US-A- 6 019 956** |

- **M. L. OCCELLI ET AL.: "Quaternary ammonium cation effects on the crystallization of offretite-erionite type zeolites: Part 1. Synthesis and catalystic properties" ZEOLITES., vol. 7, 1987, pages 265-271, XP008043268 USELSEVIER SCIENCE PUBLISHING. cited in the application**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 742 874 B1

Printed by Jouve, 75001 PARIS (FR)

## Description

### BACKGROUND OF THE INVENTION

This invention relates to an aluminosilicate zeolitic material identified as UZM-16. The material can be dealuminated to form UZM-16HS providing materials with different acidity, porosity, and ion-exchange properties. UZM-16 and UZM-16HS are useful in hydrocarbon conversion processes.

Zeolites are crystalline aluminosilicate compositions which are microporous and which are formed from corner sharing $AlO_2$ and $SiO_2$ tetrahedra. Numerous zeolites, both naturally occurring and synthetically prepared are used in various industrial processes. Synthetic zeolites are prepared via hydrothermal synthesis employing suitable sources of Si, Al, as well as structure directing agents such as alkali metals, alkaline earth metals, amines, or organoammonium cations. The structure directing agents reside in the pores of the zeolite and are largely responsible for the particular structure that is ultimately formed. These species balance the framework charge associated with aluminum and can also serve as space fillers. Zeolites are characterized by having pore openings of uniform dimensions, having a significant ion exchange capacity, and being capable of reversibly desorbing an adsorbed phase which is dispersed throughout the internal voids of the crystal without significantly displacing any atoms which make up the permanent zeolite crystal structure. Zeolites can be used as catalysts for hydrocarbon conversions, which can take place on outside surfaces as well as on internal surfaces within the pore.

A synthetic zeolite designated Zeolite T has been prepared in the Na, K system and disclosed by Breck and Acara in US-A-2950952. A subsequent crystallographic investigation employing electron diffraction (J. M. Bennett and J. A. Gard, Nature, 214, 1005 (1967)) provided distinctions and established the uniqueness of the offretite and erionite frameworks and classified Zeolite T as an intergrowth of the two structures. A synthetic zeolite with the offretite structure free of erionite intergrowths was prepared in the Na, K, TMA and K, TMA systems (R. Aiello and R. Barrer, J. Chem. Soc. (A), 1470, (1970)). Rubin and Rosinsky were able to prepare a material related to erionite in the benzyltrimethylammonium (BzTMA), Na, K system (US-A-3699139). Working in the same system, an offretite sample free of x-ray diffraction lines due to erionite were prepared (M. L. Occelli, R. A. Innes, S. S. Pollack, and J. V. Sanders, Zeolites, 7, 265 (1987)). An electron diffraction study of offretite, erionite, and offretite-erionite species resulting from syntheses that cover six organic template systems, including BzTMA, characterize the many types of faults that occur in this system (J. V Sanders, M. L. Occelli, R. A. Innes, and S. S. Pollack, Studies in Surface Science and Catalysis, Ed. Y. Murakami, A. Iijima, and J. W. Ward, Elsevier, New York, 28, 429, (1986)).
US-A-3,599,139 describes a synthetic aluminosilicate zeolite having a rigid three dimensional structure characterised by having benzyltrimethylammonium ions in the structure.
US 4,687,653 describes a zeolite having a composition of the following formula:

$$xM_{2/n}O.\ Al_2O_3.\ (5\ \text{-}10)\ SiO_2\ .\ yH_2O$$

wherein M is at least one cation having a valency of n and x is 0.8-2 and y is 0-10 and exhibiting an X-ray powder diffraction pattern shown in Table 1.

Applicants have now prepared a zeolite designated UZM-16 which has some similarities to offretite, but has sufficient differences rendering it a unique new structure. UZM-16 can be prepared in the benzyltrimethylammonium (BzTMA) system with a stall amount of additional potassium. The x-ray diffraction pattern is similar to offretite, lacking the lines associated with erionite, but also includes a broad line in the pattern centered at d = 21.5 Å, that includes unresolved peaks extending from d = 18 Å to 27 Å. This feature is observed in the electron diffraction patterns of the crystals and as fringes in high resolution lattice images. In addition, a unique periodicity observed in the a-b plane that is not known for offretite and erionite is observed in the electron diffraction patterns of UZM-16. UZM-16 has more mesoporous character than offretite.

### BRIEF DESCRIPTION OF THE DRAWINGS

Figure 1 presents electron diffraction patterns of offretite (Fig. 1A) and UZM-16 (Fig. 1B) from example 1, each taken along [110].

Figure 2 is a lattice image obtained by High Resolution Electron Microscopy of UZM-16 from example 1.

### DETAILED DESCRIPTION OF THE INVENTION

In the first aspect of the present invention there is provided a microporous crystalline zeolite as defined in claim 1.

In the second aspect of the present invention there is provided a process for preparing the microporous crystalline zeolite as defined in claim 1 by the process defined in claim 3.

In the third aspect of the present invention there is provided a process for preparing a microporous crystalline zeolite, the process being defined in claim 4.

Applicants have synthesized a new family of zeolites designated UZM-16. In its as-synthesized form, the UZM-16 zeolite has a composition on an anhydrous basis that is represented by the formula:

$$M_m^{n+} R_r^{p+} Al_{(1-x)} E_x Si_y O_z \qquad (1)$$

where M is an exchangeable cation and is selected from the group consisting of alkali and alkaline earth metals. Specific examples of the M cations include but are not limited to lithium, sodium, potassium, cesium, strontium, calcium, magnesium, barium and mixtures thereof, but potassium and sodium are preferred. The value of "m" which is the mole ratio of M to (Al + E) varies from 0 to 0.75. R is benzyltrimethylammonium (BzTMA) cation or a combination of BzTMA and at least one organoammonium cation selected from the group consisting of quaternary ammonium cations, protonated amines, protonated diamines, protonated alkanolamines, diquaternary ammonium cations, quaternized alkanolammonium cations and mixtures thereof. The value of "r" which is the mole ratio of R to (Al + E) varies from 0.25 to 5.0. The value of "n" which is the weighted average valence of M varies from +1 to +2. The value of "p", which is the average weighted valence of the organic cation has a value from +1 to +2. E is an element which is present in the framework and is selected from the group consisting of gallium, iron, boron, chromium, indium and mixtures thereof. The value of "x" which is the mole fraction of E varies from 0 to 1.0. The ratio of silicon to (Al+E) is represented by "y" which varies from greater than 3 to 25, while the mole ratio of O to (Al+E) is represented by "z" and has a value given by the equation:

$$z = (m \bullet n + r \bullet p + 3 + 4 \bullet y)/2.$$

When M is only one metal, then the weighted average valence is the valence of that one metal, i.e. +1 or +2. However, when more than one M metal is present, the total amount of:

$$M_m^{n+} = M_{m1}^{(n1)+} + M_{m2}^{(n2)+} + M_{m3}^{(n3)+} + \ldots\ldots$$

and the weighted average valence "n" is given by the equation:

$$n = \frac{m_1 \bullet n_1 + m_2 \bullet n_2 + m_3 \bullet n_3 + \cdots}{m_1 + m_2 + m_3 \cdots}$$

Similarly when only one R organic cation is present, the weighted average valence is the valence of the single R cation, i.e., +1 or +2. When more than one R cation, is present, the total amount of R is given by the equation:

$$R_r^{p+} = R_{r1}^{(p1)+} + R_{r2}^{(p2)+} + R_{r3}^{(p3)+}$$

and the weighted average valence "p" is given by the equation:

$$p = \frac{p_1 \bullet r_1 + p_2 \bullet r_2 + p_3 \bullet r_3 + \cdots}{r_1 + r_2 + r_3 + \cdots}.$$

These aluminosilicate zeolites, are prepared by a hydrothermal crystallization of a reaction mixture prepared by combining reactive sources of R, aluminum, optionally E and/or M and silicon in aqueous media. Accordingly, the aluminum sources include, but are not limited to, aluminum alkoxides, precipitated alumina, aluminum hydroxide, aluminum salts and aluminum metal. Specific examples of aluminum alkoxides include, but are not limited to aluminum

orthosec-butoxide, and aluminum orthoisopropoxide. Sources of silica include but are not limited to tetraethylorthosilicate, fumed silicas, precipitated silicas and colloidal silica. A preferred source of silica is Ultrasil VN SP (89% $SiO_2$). Sources of the M metals include but are not limited to the halide salts, nitrate salts, acetate salts, and hydroxides of the respective alkali or alkaline earth metals. In particular, M metals may occur in impurities in some organoammonium hydroxide sources as well as in some silica sources. For instance, Ludox AS-40 colloidal silica can contain on the order of 0.05% Na while Benzyltrimethylammoniumhydroxide from Aldrich Chemical Company is contaminated with on the order of 0.5 % K. Sources of the E elements include but are not limited to alkali borates, boric acid, precipitated gallium oxyhydroxide, gallium sulfate, ferric sulfate, ferric chloride, chromium chloride, chromium nitrate, indium chloride and indium nitrate. As stated when R is Benzyltrimethylammonium the sources include but are not limited to the hydroxide, chloride, bromide, iodide, and fluoride compounds. R may also be a combination of benzyltrimethylammonium and at least one other organoammonium compound. In the case where R is a quaternary ammonium cation or a quaternized alkanolammonium cation, the sources can be the hydroxide, chloride, bromide, iodide and fluoride compounds. Specific examples include without limitation diethyldimethylammonium hydroxide, tetraethylammonium hydroxide, hexamethonium bromide, tetramethylammonium chloride, methyltriethylammonium hydroxide. The source of R may also be neutral amines, diamines, and alkanolamines. Specific examples are triethanolamine, triethylamine, and N,N,N',N'tetramethyl-1,6-hexanediamine. In a special case, a reagent in the form of an aluminosilicate stock solution may be used. These solutions consist of one or more organoammonium hydroxides and sources of silicon and aluminum that are processed to form a clear homogenous solution that is generally stored and used as a reagent. The reagent contains aluminosilicate species that typically don't show up in zeolite reaction mixtures derived from separate sources of silicon and aluminum. The reagent is generally alkali-free or contains alkali at impurity levels from the silicon, aluminum, and organoammonium hydroxide sources. One or more of these solutions may be used in a zeolite synthesis. In the case of substitution of Al by E, the corresponding metallosilicate solution may also be employed in a synthesis.

The reaction mixture containing reactive sources of the desired components can be described in terms of molar ratios of the oxides by the formula:

$$aM_{2/n}O:bR_{2/p}O:(1-c)Al_2O_3:cE_2O_3:dSiO_2:eH_2O$$

where "a" is the mole ratio of the oxide of M and has a value of 0 to 5, "b" is the mole ratio of the oxide of R and has a value of 1 to 120, "d" is the mole ratio of silica and has a value of 5 to 100, "c" is the mole ratio of the oxide of E and has a value from 0 to 1.0, and "e" is the mole ratio of water and has a value of 50 to 15000. The reaction mixture is now reacted at reaction conditions including a temperature of 80°C to 160°C and preferably from 95°C to 125°C for a period of 2 days to 30 days and preferably for a time of 5 days to 15 days in a sealed reaction vessel under autogenous pressure. After crystallization is complete, the solid product is isolated from the heterogeneous mixture by means such as filtration or centrifugation, and then washed with de-ionized water and dried in air at ambient temperature up to 100°C.

The crystalline zeolites are characterized by a three-dimensional framework structure of at least $SiO_2$ and $AlO_2$ tetrahedral units. These zeolites are further characterized by their x-ray diffraction pattern. The x-ray diffraction pattern has at least the diffraction lines with the *d*-spacings and relative intensities listed in Table A.

Table A

| 2-θ | d(Å) | $I/I_0\%$ |
|---|---|---|
| 3.86-4.22 | 22.87 - 20.92 | w-m |
| 7.60-7.84 | 11.62 - 11.27 | s-vs |
| 11.58-11.86 | 7.64-7.46 | w-m |
| 13.29-13.54 | 6.65-6.53 | m |
| 13.90-14.20 | 6.36-6.23 | w |
| 15.34-15.68 | 5.77 - 5.65 | m |
| 19.30-19.65 | 4.60-4.51 | m |
| 20.37-20.73 | 4.35-4.28 | m-s |
| 23.18-23.54 | 3.83-3.78 | m-s |
| 23.57-23.89 | 3.77-3.72 | s-vs |
| 24.68-25.03 | 3.60 - 3.55 | m-s |
| 26.84-27.23 | 3.32 - 3.27 | m |

(continued)

| 2-θ | d(Å) | $I/I_0$% |
|---|---|---|
| 28.15-28.58 | 3.17-3.12 | m |
| 31.25-31.71 | 2.86 - 2.82 | vs |
| 33.37-33.76 | 2.68 - 2.65 | w |
| 35.89-36.36 | 2.50 - 2.47 | m |
| 48.05-48.52 | 1.89 - 1.87 | w-m |
| 51.38-51.90 | 1.78 - 1.76 | w-m |
| 55.35-56.04 | 1.66 - 1.64 | w-m |
| 58.08-58.64 | 1.59 - 1.57 | w |

As-synthesized, the zeolites will contain some of the exchangeable or charge balancing cations in its pores. These exchangeable cations can be exchanged for other cations, or in the case of organic cations, they can be removed by heating under controlled conditions. Ion exchange involves contacting the zeolites with a solution containing the desired cation (at molar excess) at exchange conditions. Exchange conditions include a temperature of 15°C to 100°C and a time of 20 minutes to 50 hours. Calcination conditions include a temperature of 300°C to 600°C for a time of 2 to 24 hours.

A special treatment for removing organic cations which provides the ammonium form of the zeolite is ammonia calcination. Calcination in an ammonia atmosphere can decompose organic cations, presumably to a proton form that can be neutralized by ammonia to form the ammonium cation. The resulting ammonium form of the zeolite can be further ion-exchanged to any other desired form. Ammonia calcination conditions include treatment in the ammonia atmosphere at temperatures between 250°C and 600°C and more preferably between 250°C and 450°C for times of 10 minutes to 5 hours. Optionally, the treatments can be carried out in multiple steps within this temperature range such that the total time in the ammonia atmosphere does not exceed 5 hours. Above 500°C, the treatments should be brief, less than a half hour and more preferably on the order of 5-10 minutes. Extended calcination times above 500°C can lead to unintended dealumination along with the desired ammonium ion-exchange and are unnecessarily harsh as most organoammonium templates easily decompose at lower temperatures.

The ion exchanged form of UZM-16 can be described by the empirical formula:

$$M'^{n'+}_{m'} R^{p+}_{r'} Al_{(1-x)} E_x Si_y O_{z'} \qquad (2)$$

where R, x, y, and E are as described above and m' has a value from 0 to 5.75, M' is a cation selected from the group consisting of alkali metals, alkaline earth metals, rare earth metals, hydrogen ion, ammonium ion, and mixtures thereof, n' is the weighted average valence of M' and varies from 1 to 3, r' has a value from 0 to 5.75, r' + m' > 0, and p is the weighted average valence of R and varies from +1 to +2. The value of z' is given by the formula:

$$z' = (m' \bullet n' + r' \bullet p + 3 + 4 \bullet y)/2.$$

The UZM-16 zeolites represented by equation (2) can be further treated in order to remove aluminum and optionally insert silicon thereby increasing the Si/Al ratio and thus modifying the acidity and ion exchange properties of the zeolites. These treatments include: a) contacting with a fluorosilicate solution or slurry; b) calcining or steaming followed by acid extraction or ion-exchange; c) acidextraction or d) any combination of these treatments in any order.

Fluorosilicate treatment is known in the art and is described in US-A-6,200,463 B1, which cites US-A-4,711,770 as describing a process for treating a zeolite with a fluorosilicate salt. General conditions for this treatment are contacting the zeolite with a solution containing a fluorosilicate salt such as ammonium fluorosilicate (AFS) at a temperature of 20°C to 90°C.

The acids which can be used in carrying out acid extraction include without limitation mineral acids, carboxylic acids and mixtures thereof. Examples of these include sulfuric acid, nitric acid, ethylenediaminetetraacetic acid (EDTA), citric acid, oxalic acid, etc. The concentration of acid which can be used is not critical but is conveniently between 1 wt.% to 80 wt.% acid and preferably between 5 wt.% and 40 wt.% acid. Acid extraction conditions include a temperature

of 10°C to 100°C for a time of 10 minutes to 24 hours. Once treated with the acid, the treated UZM-16 zeolite is isolated by means such as filtration, washed with deionized water and dried at ' ambient temperature up to 100°C. The UZM-16 zeolites which have undergone one or more treatments whereby aluminum has been removed and optionally silicon has been inserted into the framework will hereinafter be referred to as UZM-16HS.

The extent of dealumination obtained from acid extraction depends on the cation form of the starting UZM-16 as well as the acid concentration and the time and temperature over which the extraction is conducted. For example, if organic cations are present in the starting UZM-16, the extent of dealumination will be slight compared to a UZM-16 in which the organic cations have been removed. This may be preferred if it is desired to have dealumination just at the surface of the UZM-16. As stated above, convenient ways of removing the organic cations include calcination, ammonia calcination, steaming and ion exchange. Calcination, ammonia calcination and ion exchange conditions are as set forth above. Steaming conditions include a temperature of 400°C to 850°C with from 1% to 100% steam for a time of 10 minutes to 48 hours and preferably a temperature of 500°C to 600°C, steam concentration of 5 to 50% and a time of 1 to 2 hours.

It should be pointed out that both calcination and steaming treatments not only remove organic cations, but can also dealuminate the zeolite. Thus, alternate embodiments for dealumination include: a calcination treatment followed by acid extraction and steaming followed by acid extraction. A further embodiment for dealumination comprises calcining or steaming the starting UZM-16 zeolite followed by an ion-exchange treatment. Of course an acid extraction can be carried out concurrently with, before or after the ion exchange.

The ion exchange conditions are the same as set forth above, namely a temperature of 15°C to 100°C and a time of 20 minutes to 50 hours. Ion exchange can be carried out with a solution comprising a cation (M1') selected from the group consisting of alkali metals, alkaline earth metals, rare earth metals, hydrogen ion, ammonium ion, and mixtures thereof. By carrying out this ion exchange, the M1 cation is exchanged for a secondary or different M1' cation. In a preferred embodiment, the UZM-16HS composition after the steaming or calcining steps is contacted with an ion exchange solution comprising an ammonium salt. Examples of ammonium salts include but are not limited to ammonium nitrate, ammonium chloride, ammonium bromide, and ammonium acetate. The ammonium ion containing solution can optionally contain a mineral acid such as but not limited to nitric, hydrochloric, sulfuric and mixtures thereof. The concentration of the mineral acid is that amount necessary to give a ratio of $H^+$ to $NH_4^+$ of 0 to 1. This ammonium ion exchange aids in removing any debris present in the pores after the steaming and/or calcination treatments.

It is apparent from the foregoing that, with respect to effective process conditions, it is desirable that the integrity of the zeolite crystal structure be substantially maintained throughout the dealumination process, and that the zeolite retains at least 50%, preferably at least 70% and more preferably at least 90% of its original crystallinity. A convenient technique for assessing the crystallinity of the products relative to the crystallinity of the starting material is the comparison of the relative intensities of the *d*-spacing of their respective X-ray powder diffraction patterns. The sum of the peak intensities, in arbitrary units above the background, of the starting material is used as the standard and is compared with the corresponding peak intensities of the products. When, for example, the numerical sum of the peak heights of the molecular sieve product is 85 percent of the value of the sum of the peak intensities of the starting zeolite, then 85 percent of the crystallinity has been retained. In practice it is common to utilize only a portion of the peaks for this purpose, as for example, five or six of the strongest peaks. Other indications of the retention of crystallinity are surface area and adsorption capacity. These tests may be preferred when the substituted metal significantly changes, e.g., increases, the absorption of x-rays by the sample or when peaks experience substantial shifts such as in the dealumination process.

After having undergone any of the dealumination treatments as described above, the UZM-16HS is usually dried and can be used in various processes as discussed below. Applicants have found the properties of the UZM-16HS can be further modified by one or more additional treatment. These treatments include steaming, calcining or ion exchanging and can be carried out individually or in any combination. Some of these combinations include but are not limited to:

steam → calcine → ion exchange;

calcine → steam → ion exchange;

ion exchange → calcine → steam

ion exchange → steam → calcine;

steam → calcine;

The dealumination treatment described above can be combined in any order to provide the zeolites of the invention although not necessarily with equivalent result. It should be pointed out that the particular sequence of treatments, e.g., AFS, acid extraction, steaming, calcining, etc can be repeated as many times as necessary to obtain the desired properties. Of course one treatment can be repeated while not repeating other treatments, e.g., repeating the AFS two or more times before carrying out steaming or calcining; etc. Finally, the sequence and/or repetition of treatments will determine the properties of the final UZM-16HS composition.

The UZM-16HS as prepared above is described by the empirical formula on an anhydrous basis of

$$M1_a^{n+}Al_{(1-x)}E_xSi_{y'}O_{z''} \quad (3)$$

where M1 is at least one exchangeable cation selected from the group consisting of alkali, alkaline earth metals, rare earth metals, ammonium ion, hydrogen ion and mixtures thereof, a is the mole ratio of M1 to (A1 + E) and varies from 0.01 to 50, n is the weighted average valence of M1 and has a value of +1 to +3, E is an element selected from the group consisting of gallium, iron, boron, chromium, indium and mixtures thereof, x is the mole fraction of E and varies from 0 to 1.0, y' is the mole ratio of Si to (A1 + E) and varies from greater than 3 to virtually (pure silica) and z" is the mole ratio of O to (Al + E) and has a value determined by the equation:

$$z'' = (a \bullet n + 3 + 4 \bullet y')/2;$$

the zeolite characterized in that it has an x-ray diffraction pattern having at least the d-spacings and relative intensities set forth in Table B.

Table B

| 2-θ | d(Å) | $I/I_0$% |
|---|---|---|
| 7.70 - 8.40 | 11.47 - 10.52 | m-vs |
| 11.70 - 12.10 | 7.56 - 7.31 | w |
| 13.35 - 14.56 | 6.63 - 6.08 | s - vs |
| 20.60 - 21.70 | 4.31 - 4.09 | w |
| 24.60 - 25.65 | 3.62 - 3.47 | m-s |

By virtually pure silica is meant that virtually all the aluminum and/or the E metals have been removed from the framework. It is well know that it is virtually impossible to remove all the aluminum and/or E metal. Numerically, a zeolite is virtually pure silica when y' has a value of at least 3,000, preferably 10,000 and most preferably 20,000. Thus, ranges for y' are from 3 to 3,000 preferably greater than 10 to 3,000; 3 to 10,000 preferably greater than 10 to 10,000 and 3 to 20,000 preferably greater than 10 to 20,000.

In specifying the proportions of the zeolite starting material or adsorption properties of the zeolite product and the like herein, the "anhydrous state" of the zeolite will be intended unless otherwise stated. The term "anhydrous state" is employed herein to refer to a zeolite substantially devoid of both physically adsorbed and chemically adsorbed water.

The zeolites of this invention (both UZM-16 and UZM-16HS) are capable of separating mixtures of molecular species based on the molecular size (kinetic diameter) or on the degree of polarity of the molecular species. When the separation of molecular species is based on molecular size, separation is accomplished by the smaller molecular species entering the intracrystalline void space while excluding larger species. The kinetic diameters of various molecules such as oxygen, nitrogen, carbon dioxide, carbon monoxide are provided in D.W. Breck, Zeolite Molecular Sieves, John Wiley and Sons (1974) p. 636.

The crystalline microporous compositions of the present invention (both UZM-16 and UZM-16HS) either as-synthesized, after calcination or after any of the above referenced treatments can be used as catalysts or catalyst supports in hydrocarbon conversion processes. Hydrocarbon conversion processes are well known in the art and include ring-opening, cracking, hydrocracking, alkylation of both aromatics and isoparaffins, isomerization, polymerization, reforming, dewaxing, hydrogenation, dehydrogenation, transalkylation, dealkylation, hydration, dehydration, hydrotreating, hydrodenitrogenation, hydrodesulfurization, methanation and syngas shift process. Specific reaction conditions and the types of feeds which can be used in these processes are set forth in US-A-4,310,440 and US-A-4,440,871. A preferred hydrocarbon conversion process is ring-opening, whereby cyclic hydrocarbons are converted to noncyclic hydrocarbons,

i.e. linear or branched hydrocarbons.

Other reactions may be catalyzed by these crystalline microporous compositions, including base-catalyzed side chain alkylation of alkylaromatics, aldolcondensations, olefin double bond isomerization and isomerization of acetylenes, alcohol dehydrogenation, and olefin dimerization, oligomerization and conversion of alcohol to olefins. Suitably ion exchanged forms of these materials can catalyze the reduction of $NO_x$ to $N_2$ in automotive and industrial exhaust streams. Some of the reaction conditions and types of feeds that can be used in these processes are set forth in US-A-5,015,796 and in H. Pines, THE CHEMISTRY OF CATALYTIC HYDROCARBON CONVERSIONS, Academic Press (1981) pp. 123-154 and references contained therein.

The X-ray patterns presented in the following examples (and tables above) were obtained using standard X-ray powder diffraction techniques. The radiation source was a high-intensity X-ray tube operated at 45 kV and 35 ma. The diffraction pattern from the copper K-alpha radiation was obtained by appropriate computer based techniques. Flat compressed powder samples were continuously scanned at 2° (2θ) per minute from 2° to 70°(2θ). Interplanar spacings (d) in Angstrom units were obtained from the position of the diffraction peaks expressed as 2θ where θ is the Bragg angle as observed from digitized data. Intensities were determined from the integrated area of diffraction peaks after subtracting background, "$I_o$" being the intensity of the strongest line or peak, and "I" being the intensity of each of the other peaks.

As will be understood by those skilled in the art, the determination of the parameter 2θ is subject to both human and mechanical error, which in combination can impose an uncertainty of ±0.4 on each reported value of 2θ and up to ±0.5 on reported values for nanocrystalline materials. This uncertainty is, of course, also manifested in the reported values of the d-spacings, which are calculated from the θ values. This imprecision is general throughout the art and is not sufficient to preclude the differentiation of the present crystalline materials from each other and from the compositions of the prior art. In some of the X-ray patterns reported, the relative intensities of the d-spacings are indicated by the notations vs, s, m and w which represent very strong, strong, medium, and weak, respectively. In terms of 100 X $I/I_o$, the above designations are defined as w = 0-15; m = 15-60; s = 60-80 and vs = 80-100. In certain instances the purity of a synthesized product may be assessed with reference to its X-ray powder diffraction pattern. Thus, for example, if a sample is stated to be pure, it is intended only that the X-ray pattern of the sample is free of lines attributable to crystalline impurities, not that there are no amorphous materials present.

In order to more fully illustrate the invention, the following examples are set forth. It is to be understood that the examples are only by way of illustration and are not intended as an undue limitation on the broad scope of the invention as set forth in the appended claims.

EXAMPLE 1

An aluminosilicate reaction mixture was prepared by adding 2.33 g of $Al(Osec\text{-}Bu)_3$ (95+%) to 63.35 g BzTMAOH (40%) with vigorous stirring, followed by the addition of 28 g of colloidal silica (LUDOX™ AS-40, 40% $SiO_2$). Next 6 g of water was added slowly to the aluminosilicate reaction mixture with continued mixing. The mixture was homogenized for an additional 30 minutes with a high-speed stirrer and distributed to three teflon-lined autoclaves which were placed in a 125°C oven and the mixtures digested for 3, 6, and 10 days at autogenous pressures. The solid products were isolated by filtration, washed with deionized water, and dried at 95°C.

The products from all three of the reactions exhibited an x-ray diffraction pattern for the zeolite designated UZM-16. Representative diffraction lines from the 3-day sample are shown in Table 1 below. The BET surface area of the calcined material determined from $N_2$ adsorption was 523 $m^2/g$ and the micropore volume was0.26 cc/g.

Table 1

| 2-θ | d(Å) | $I/I_0$% |
|---|---|---|
| 4.10 | 21.49 | m |
| 7.68 | 11.50 | s |
| 11.66 | 7.58 | w |
| 13.38 | 6.61 | m |
| 14.00 | 6.32 | w |
| 15.48 | 5.72 | m |
| 19.46 | 4.56 | m |
| 20.56 | 4.32 | s |

(continued)

| 2-θ | d(Å) | $I/I_0$% |
|---|---|---|
| 23.36 | 3.80 | s |
| 23.72 | 3.75 | vs |
| 24.80 | 3.59 | s |
| 27.02 | 3.30 | m |
| 28.32 | 3.15 | m |
| 30.65 | 2.91 | w |
| 31.42 | 2.84 | vs |
| 33.52 | 2.67 | w |
| 36.08 | 2.49 | m |
| 41.00 | 2.20 | w |
| 42.92 | 2.11 | w |
| 43.68 | 2.07 | w |
| 45.92 | 1.97 | w |
| 48.28 | 1.88 | m |
| 49.86 | 1.83 | w |
| 51.62 | 1.77 | w |
| 54.14 | 1.69 | w |
| 55.90 | 1.64 | m |
| 58.42 | 1.58 | w |
| 61.69 | 1.50 | w |
| 63.76 | 1.46 | w |
| 65.40 | 1.43 | w |
| 69.00 | 1.36 | w |

The distinction of UZM-16 from the offretite structure is further distinguished by electron diffraction. Figure 1 shows a comparison of UZM-16 (Fig. 1B) and offretite (Fig. 1A) electron diffraction patterns taken along [110], shown with the (001) and the (1 10) directions in the plane as indicated. It is clear that the periodicity in the (1 10) direction in UZM-16 is different from that of offretite and that the spacing is larger by a factor of 3. The electron diffraction pattern for offretite presented here is consistent with those shown in the literature for the same orientation (J. V Sanders, M. L. Occelli, R. A. Innes, and S. S. Pollack, Studies in Surface Science and Catalysis, Ed. Y. Murakami, A. Iijima, and J. W. Ward, Elsevier, New York, 28, 429, (1986)).

The larger spacing is easily observed in the lattice images of UZM-16. Figure 2 shows the lattice image of UZM-16 taken along [110]. The lattice fringes running vertically in the image correspond to the 19.3 Å species indicated by the reflection at 1/3($1\bar{1}0$) in figure 1b. This spacing is triple that for the ($1\bar{1}0$) expected in OFF, which would be 6.65 Å.

EXAMPLE 2

This example demonstrates the preparation of UZM-16, its modification by calcination, ion-exchange and steaming to form UZM-16HS, followed by an additional modification by acid extraction to form yet another variant of UZM-16HS. An aluminosilicate reaction mixture was prepared by adding 35.21 g of AKOSeC-Bu$)_3$ (95+%) to 927.47 g BzTMAOH (40%) with vigorous stirring. After the aluminum reagent was dissolved, 416.45 g colloidal silica (LUDOX AS-40, 40% $SiO_2$) was added and the resulting mixture was homogenized for 30 minutes with a high-speed stirrer. The mixture was crystallized at 125°C for 6 days in a 2-L PARR™ stirred reactor at autogenous pressure. The solid products were isolated by filtration, washed with de-ionized water, and dried at 95°C. Powder x-ray diffraction identified the product as UZM-16. Diffraction lines characteristic of the pattern are given in Table 2 below. Elemental analysis showed this parent

UZM-16 material to have a Si/Al ratio of 5.83. A 62.5 g portion of the UZM-16 was initially calcined in a flowing nitrogen atmosphere ramping to 250°C at 1°C/min, dwelling at 250°C for 2 hr, ramping to 500°C at 1°C/min, dwelling at 500°C for 3 hr, switching the atmosphere to flowing air, and dwelling for an additional 3 hr at 500°C. The BET surface area of the calcined material was 676 $m^2$/g and the micropore volume was 0.33 cc/g. A 51 g portion of the calcined material was ammonium exchanged by suspending it in a solution containing 52 g $NH_4NO_3$ dissolved in 500 g de-ionized water. The slurry was heated to 85°C for 7 hr and then isolated by filtration and washed thoroughly with de-ionized water. The exchange was repeated two more times, but only for 2.5 hr at 85°C.

A 40 g portion of the ammonium exchanged calcined UZM-16 was steamed at 600°C for 2 hrs with 50% steam using a horizontal steamer. The product was identified as UZM-16HS via powder x-ray diffraction. Characteristic diffraction lines for this product is shown below in Table 2. Nitrogen adsorption measurements yielded a BET surface area of 424 $m^2$/g and a micropore volume of 0.20 cc/g for the steamed ammonium-exchanged calcined UZM-16HS material.

A 20 g portion of the UZM-16HS obtained via steaming was further treated with acid. An acidic solution was prepared by diluting 19.7g $HNO_3$ (69%) in 350 g deionized water. The solution was heated to 90°C before the addition of the steamed UZM-16. The resulting slurry was stirred for 1 hr at 90°C. The product was isolated by filtration, washed with de-ionized water, and dried at 98°C. The modified product was determined to be UZM-16HS via x-ray powder diffraction analysis. Characteristic diffraction lines for the product are listed in Table 2 below. Elemental analyses of the steamed/acid wash showed the product to have a Si/Al ratio of 14.76.

Table 2

| UZM-16 | | | UZM-16HS (steamed ammonium-exchanged calcined UZM-16) | | | UZM-16HS (Acid extracted steamed ammonium-exchanged calcined UZM-16) | | |
|---|---|---|---|---|---|---|---|---|
| 2-θ | d(Å) | $I/I_0$% | 2-θ | d(Å) | $I/I_0$% | 2-θ | d(Å) | $I/I_0$% |
| 4.04 | 21.85 | m | | | | | | |
| 7.72 | 11.44 | vs | 7.94 | 11.13 | vs | 8.18 | 10.80 | m |
| 11.72 | 7.55 | w | 11.86 | 7.46 | w | 11.96 | 7.39 | w |
| 13.40 | 6.60 | m | 13.64 | 6.48 | s | | | |
| 14.04 | 6.30 | w | 14.30 | 6.19 | m | 14.26 | 6.21 | vs |
| 15.52 | 5.71 | m | | | | | | |
| 19.48 | 4.55 | m | | | | 19.188 | 4.62 | w |
| 20.58 | 4.31 | s | 20.98 | 4.23 | w | 21.32 | 4.16 | w |
| 23.38 | 3.80 | m | 23.67 | 3.76 | w | | | |
| 23.76 | 3.74 | vs | 23.98 | 3.71 | w | 24.34 | 3.65 | w |
| 24.86 | 3.58 | s | 25.02 | 3.56 | m | 25.25 | 3.52 | m |
| 27.06 | 3.29 | m | | | | | | |
| 28.34 | 3.15 | m | | | | | | |
| 30.62 | 2.92 | w | | | | | | |
| 31.46 | 2.84 | vs | 31.84 | 2.81 | m | | | |
| 33.56 | 2.67 | w | | | | | | |
| 36.08 | 2.49 | m | | | | | | |
| 41.08 | 2.20 | w | | | | | | |
| 42.92 | 2.11 | w | | | | | | |
| 43.60 | 2.07 | w | | | | | | |
| 45.88 | 1.98 | w | | | | | | |
| 48.26 | 1.88 | m | | | | | | |
| 48.99 | 1.86 | w | | | | | | |
| 49.92 | 1.83 | w | | | | | | |

(continued)

| UZM-16 | | | UZM-16HS (steamed ammonium-exchanged calcined UZM-16) | | | UZM-16HS (Acid extracted steamed ammonium-exchanged calcined UZM-16) | | |
|---|---|---|---|---|---|---|---|---|
| 2-θ | d(Å) | $I/I_0$% | 2-θ | d(Å) | $I/I_0$% | 2-θ | d(Å) | $I/I_0$% |
| 51.64 | 1.77 | m | | | | | | |
| 54.14 | 1.69 | w | | | | | | |
| 55.80 | 1.65 | m | | | | | | |
| 58.40 | 1.58 | w | | | | | | |
| 61.66 | 1.50 | w | | | | | | |
| 63.68 | 1.46 | w | | | | | | |
| 65.52 | 1.42 | w | | | | | | |
| 67.99 | 1.38 | w | | | | | | |
| 68.91 | 1.36 | w | | | | | | |

EXAMPLE 3

An aluminosilicate reaction mixture was prepared by adding 35.21 g of Al(Osec-Bu)$_3$ (95+%) to 927.47 g BzTMAOH (40%) with vigorous stirring. After the aluminum reagent was dissolved, 416.45 g colloidal silica (LUDO AS-40, 40% $SiO_2$) was added and the resulting mixture was homogenized for 30 minutes with a high-speed stirrer. The mixture was crystallized at 105°C for 12 days in a 2-L PARR™ stirred reactor at autogenous pressure. The solid product was isolated by filtration, washed with deionized water, and dried at 95°C. Powder x-ray diffraction identified the product as UZM-16. Diffraction lines characteristic of the pattern are given in Table 3 below. Elemental analysis showed this parent UZM-16 composition to have the elemental mole ratio Si/Al = 5.7. The product was calcined initially in a nitrogen atmosphere ramping to 350°C at 1°C/min, dwelling at 350°C for 1 hr, ramping to 500°C at 1°C/min, dwelling at 500°C for 6 hr, switching the atmosphere to air, and dwelling for an additional 6 hr at 500°C. The BET surface area of the calcined material was 574 $m^2$/g and the micropore volume was 0.24 cc/g. A 98 g portion of the calcined product was ammonium exchanged by suspending it in a solution containing 100 g $NH_4NO_3$ dissolved in 1000 ml deionized water. The slurry was heated for 5 hr at 80°C, the product isolated by filtration and washed thoroughly with de-ionized water. This exchange was repeated 3 additional times.

An 18 g portion of the ammonium exchanged calcined UZM-16 was steamed at 600°C for 2 hrs with 50% steam using a horizontal steamer. A 12 g portion of this steamed material was additionally acid extracted. An acidic solution was prepared by diluting 10 g $HNO_3$ (69%) in 300 g de-ionized water. The solution was heated to 90°C before the addition of the steamed ammonium-exchanged calcined UZM-16. The resulting slurry was stirred for 1 hr at 90°C. The product was isolated by filtration, washed with de-ionized water, and dried at 98°C. The modified product was identified as UZM-16HS via x-ray powder diffraction. Characteristic diffraction lines for the product are listed in Table 3. Elemental analyses showed the final dealuminated product to have a Si/Al ratio of 11.56. Nitrogen adsorption measurements showed the BET surface area to be 466 $m^2$/g, while the micropore volume was 0.19 cc/g.

Table 3

| UZM-16 | | | UZM-16HS (acid extracted steamed ammonium-exchanged calcined UZM-16) | | |
|---|---|---|---|---|---|
| 2-θ | d(Å) | $I/I_0$% | 2-θ | d(Å) | $I/I_0$% |
| 3.93 | 22.49 | w | | | |
| 7.66 | 11.53 | s | 8.08 | 10.93 | m |
| 11.70 | 7.56 | w | 11.84 | 7.47 | w |
| 13.38 | 6.61 | m | 13.84 | 6.39 | vs |
| 14.00 | 6.32 | w | | | |
| 15.44 | 5.73 | m | | | |
| 19.44 | 4.56 | m | 19.88 | 4.46 | w |

(continued)

| UZM-16 | | | UZM-16HS (acid extracted steamed ammonium-exchanged calcined UZM-16) | | |
|---|---|---|---|---|---|
| 2-θ | d(Å) | $I/I_0$% | 2-θ | d(Å) | $I/I_0$% |
| 20.56 | 4.32 | m | 21.08 | 4.21 | w |
| 23.32 | 3.81 | s | | | |
| 23.76 | 3.74 | s | | | |
| 24.82 | 3.58 | vs | 25.06 | 3.55 | m |
| 27.05 | 3.29 | m | | | |
| 28.32 | 3.15 | m | | | |
| 31.48 | 2.84 | vs | 31.80 | 2.81 | w |
| 33.58 | 2.67 | w | | | |
| 36.22 | 2.48 | w | | | |
| 48.24 | 1.89 | w | | | |
| 49.83 | 1.83 | w | | | |
| 51.64 | 1.77 | m | | | |
| 55.58 | 1.65 | m | | | |
| 58.34 | 1.58 | w | | | |
| 61.58 | 1.50 | w | | | |
| 63.69 | 1.46 | w | | | |
| 65.43 | 1.43 | w | | | |
| 68.02 | 1.38 | w | | | |
| 68.83 | 1.36 | w | | | |

## Claims

**1.** A microporous crystalline zeolite having a composition in the as-synthesised and anhydrous form in terms of mole ratios of the elements of:

$$M^{n+}{}_m R^{p+}{}_r Al_{(1-x)} E_x Si_y O_z$$

where M is at least one exchangeable cation selected from the group consisting of alkali and alkaline earth metals, "m" is the mole ratio of M to (Al+E) and varies from 0 to 0.75, R is benzyltrimethylammonium (BzTMA) or a combination of BzTMA and at least one organoammonium cation selected from the group consisting of quaternary ammonium cations, protonated amines, protonated diamines, protonated alkanolamines, diquaternaryammonium cations, quaternized alkanolamines and mixtures thereof, "r" is the mole ratio of R to (Al+E) and has a value of 0.25 to 5.0, E is an element selected from the group consisting of Ga, Fe, In, Cr, B, and mixtures thereof, "x" is the mole fraction of E and varies from 0 to 1.0, "n" is the weighted average valence of M and has a value of +1 to +2, "p" is the weighted average valence of R and has a value of +1 to +2, "y" is the mole ratio of Si to (Al + E) and varies from greater than 3 to 25 and "z" is the mole ratio of O to (Al+E) and has a value determined by the equation:

$$z = (m \cdot n + r \cdot p + 3 + 4 \cdot y)/2;$$

the zeolite **characterized in that** it has an x-ray diffraction pattern having at least the d-spacings and relative intensities set forth in Table A:

Table A

| 2-θ | d(A) | $I/I_0\%$ |
|---|---|---|
| 3.86-4.22 | 22.87-20.92 | w-m |
| 7.60-7.84 | 11.62-11.27 | s-vs |
| 11.58-11.86 | 7.64 - 7.46 | w-m |
| 13.29-13.54 | 6.65-6.53 | m |
| 13.90-14.20 | 6.36-6.23 | w |
| 15.34-15.68 | 5.77-5.65 | m |
| 19.30-19.65 | 4.60-4.51 | m |
| 20.37-20.73 | 4.35-4.28 | m-s |
| 23.18-23.54 | 3.83-3.78 | m-s |
| 23.57-23.89 | 3.77-3.72 | s-vs |
| 24.68-25.03 | 3.60-3.55 | m-s |
| 26.84-27.23 | 3.32-3.27 | m |
| 28.15-28.58 | 3.17-3.12 | m |
| 31.25-31.71 | 2.86-2.82 | vs |
| 33.37-33.76 | 2.68-2.65 | w |
| 35.89-36.36 | 2.50-2.47 | m |
| 48.05-48.52 | 1.89-1.87 | w-m |
| 51.38-51.90 | 1.78-1.76 | w-m |
| 55.35-56.04 | 1.66-1.64 | w-m |
| 58.08-58.64 | 1.59-1.57 | w |

**2.** The zeolite of claim 1 where "m" is zero.

**3.** A process for preparing the microporous crystalline zeolite of Claim 1 comprising forming a reaction mixture containing reactive sources of R, Al, Si and optionally E and/or M and heating the reaction mixture at a temperature of 80°C to 160°C, the reaction mixture having a composition expressed in terms of mole ratios of the oxides of:

$$aM_{2/n}O{:}bR_{2/p}O{:}(1\text{-}c)Al_2O_3{:}cE_2O_3{:}dSiO_2{:}cH_2O$$

where "a" has a value of 0 to 5.0, "b" has a value of 1 to 120, "c" has a value of 0 to 1.0, "d" has a value of 5 to 100, and "e" has a value of 50 to 15000.

**4.** A process for preparing a microporous crystalline zeolite wherein the microporous crystalline zeolite has an empirical composition on an anhydrous basis in terms of mole ratios of the elements of:

$$M1^{n+}{}_aAl_{(1-x)}E_xSi_yO_{z''}$$

where M1 is at least one exchangeable cation selected from the group consisting of alkali metals, alkaline earth metals, rare earth metals, ammonium ion, hydrogen ion and mixtures thereof, a is the mole ratio of M 1 to (Al+E) and varies from 0.01 to 50, E is an element selected from the group consisting of gallium, iron, boron, chromium, indium and mixtures therefore, x is the mole fraction of E and varies from 0 to 1.0, n is the weighted average valence of M1 and has a value of +1 to +3, y' is the mole ratio of Si to (Al+E) and is greater than 3 and z" is the mole ratio of O to (Al+E) and has a value determined by the equation:

$$z'' = (a \cdot n+3+4 \cdot y')/2;$$

the zeolite **characterized in that** it has an x-ray diffraction pattern having at least the *d*-spacings and relative intensities set forth in Table B:

Table B

| 2-θ | d(A) | $I/I_0$% |
|---|---|---|
| 7.70-8.40 | 11.47-10.52 | m-vs |
| 11.70-12.10 | 7.56-7.31 | w |
| 13.35-14.56 | 6.63-6.08 | s-vs |
| 20.60-21.70 | 4.31-4.09 | w |
| 24.60-25.65 | 3.62-3.47 | m-s |

the method comprising treating a microporous crystalline zeolite as defined in claim 1 to exchange exchangeable cations for other cations, or in the case of organic cations removing by heating under controlled conditions to form a starting zeolite, and treating the starting zeolite at treating conditions thereby removing at least a portion of the framework aluminium and optionally inserting silicon into the framework to provide the microporous crystalline zeolite; the starting zeolite having an empirical formula on an anhydrous basis of:

$$M'^{n+}_{m'} R^{p+}_{r'} Al_{(1-x)} E_x Si_{y'} O_{z'}$$

where M' is an exchangeable cation selected from the group consisting of ammonium ion, hydrogen ion, alkali metals, alkaline earth metals, rare earth metals and mixtures thereof, n is the weighted average valence of M' and varies from +1 to +3, m' is the mole ratio of M' to (Al+E) and varies from 0 to 5.75, R is benzyltrimethylammonium (BzTMA) or a combination of BzTMA and at least one organoammonium cation selected from the group consisting of protonated amines, protonated diamines, protonated alkanolamines, quaternary ammonium ions, diquatemary ammonium ions, quaternized alkanolammonium ions and mixtures thereof, p is the average weighted valence of the organic cation and varies from +1 to +2, r' is the mole ratio of R to (Al+E) and varies from 0 to 5.75, r'+m'>0, y' is the ratio of Si to (Al+E) and varies from greater than 3 to 25 and z' is the mole ratio of O to (Al+E) and has a value given by the equation:

$$z' = (m' \cdot n+r' \cdot p+3+4 \cdot y')/2,$$

the zeolite **characterized in that** it has an x-ray diffraction pattern having at least the d-spacings and relative intensities set forth in Table A.

**5.** The process of claim 4 where the treating step is selected from the group consisting of treatment with a fluorosilicate solution or slurry, extraction with a weak, strong or complexing acid, calcination plus ion-exchange, calcination plus acid extraction, steaming plus ion-exchange, steaming plus acid extraction and mixtures thereof.

**6.** A hydrocarbon conversion process comprising contacting a hydrocarbon with a catalytic composite at hydrocarbon conversion conditions to give a converted product, the catalytic composite comprising the microporous crystalline zeolite of Claim 1.

**7.** The process of claim 6 where the hydrocarbon conversion process is selected from the group consisting of alkylation of aromatics, isomerisation of xylenes, naphtha cracking, ring opening, transalkylation, alkylation of isoparaffins and isomerisation of ethyl benzene.

## Patentansprüche

1. Mikroporöser kristalliner Zeolith mit einer Zusammensetzung der wie synthetisierten und wasserfreien Form in Molanteilen der Elemente von:

$$M^{n+}{}_{m}R^{p+}{}_{r}Al\ (1\text{-}x)E_{x}Si_{y}O_{z}$$

wobei M wenigstens ein austauschbares Kation ist, ausgewählt aus der Gruppe bestehend aus Alkaliund Erdalkalimetallen, "m" das Molverhältnis von M zu (Al + E) ist und von 0 bis 0,75 variiert, R Benzyltrimethylammonium (BzTMA) oder eine Kombination von BzTMA und wenigstens einem Organoammonium-Kation, ausgewählt aus der Gruppe bestehend aus quaternären Ammonium-Kationen, protonierten Aminen, protonierten Diaminen, protonierten Alkanolaminen, diquaternären Ammonium-Kationen, quaternisierten Alkanolaminen und Gemischen davon ist, "r" das Molverhältnis von R zu (Al + E) ist und einen Wert von 0,25 bis 5,0 aufweist, E ein Element ist, ausgewählt aus der Gruppe bestehend aus Ga, Fe, In, Cr, B und Gemischen davon, "x" der Molanteil von E ist und von 0 bis 1,0 variiert, "n" die gewichtete mittlere Valenz von M ist und einen Wert von +1 bis +2 aufweist, "p" die gewichtete mittlere Valenz von R ist und einen Wert von +1 bis +2 aufweist, "y" das Molverhältnis von Si zu (Al + E) ist und von größer als 3 bis 25 variiert, und "z" das Molverhältnis von O zu (Al + E) ist und einen Wert aufweist, der durch die Gleichung:

$$z = (m\cdot n+r\cdot p+3+4\cdot y)/2;$$

bestimmt ist, wobei der Zeolith **dadurch gekennzeichnet ist, dass** er ein Röntgendiffraktionsmuster aufweist, das wenigstens die in Tabelle A aufgeführten d-Abstände und relativen Intensitäten aufweist:

Tabelle A

| 2-θ | d (Å) | $I/I_0$ % |
|---|---|---|
| 3,86-4,22 | 22,87-20,92 | w-m |
| 7,60-7,84 | 11,62-11,27 | s-vs |
| 11,58-11,86 | 7,64-7,46 | w-m |
| 13,29-13,54 | 6,65-6,53 | m |
| 13,90-14,20 | 6,36-6,23 | w |
| 15,34-15,68 | 5,77-5,65 | m |
| 19,30-19,65 | 4,60-4,51 | m |
| 20,37-20,73 | 4,35-4,28 | m-s |
| 23,18-23,54 | 3,83-3,78 | m-s |
| 23,57-23,89 | 3,77-3,72 | s-vs |
| 24,68-25,03 | 3,60-3,55 | m-s |
| 26,84-27,23 | 3,32-3,27 | m |
| 28,15-28,58 | 3,17-3,12 | m |
| 31,25-31,71 | 2,86-2,82 | vs |
| 33,37-33,76 | 2,68-2,65 | w |
| 35,89-36,36 | 2,50-2,47 | m |
| 48,05-48,52 | 1,89-1,87 | w-m |
| 51,38-51,90 | 1,78-1,76 | w-m |
| 55,35-56,04 | 1,66-1,64 | w-m |
| 58,08-58,64 | 1,59-1,57 | w |

**2.** Zeolith gemäß Anspruch 1, wobei "m" null ist.

**3.** Verfahren zum Herstellen des mikroporösen kristallinen Zeoliths gemäß Anspruch 1, umfassend das Bilden eines Reaktionsgemischs, umfassend reaktionsfähige Quellen von R, Al, Si und gegebenenfalls E und/oder M, und Erhitzen des Reaktionsgemischs auf eine Temperatur von 80 °C bis 160 °C, wobei das Reaktionsgemisch eine Zusammensetzung, ausgedrückt als Molverhältnisse der Oxide, von:

$$aM_{2/n}O:bR_{2/p}O : (1\text{-}c)Al_2O_3:cE_2O_3:dSiO_2:eH_2O$$

aufweist, wobei "a" einen Wert von 0 bis 5,0 aufweist, "b" einen Wert von 1 bis 120 aufweist, "c" einen Wert von 0 bis 1,0 aufweist, "d" einen Wert von 5 bis 100 aufweist und "e" einen Wert von 50 bis 15000 aufweist.

**4.** Verfahren zum Herstellen eines mikroporösen kristallinen Zeoliths, wobei der mikroporöse kristalline Zeolith eine empirische Zusammensetzung auf wasserfreier Basis in Molanteilen der Elemente von:

$$\boldsymbol{M1^{n+}{}_{a}Al_{(1-x)}E_{x}Si_{y'}O_{z''}}$$

aufweist, wobei M1 wenigstens ein austauschbares Kation ist, ausgewählt aus der Gruppe bestehend aus Alkalimetallen, Erdalkalimetallen, Seltenerdmetallen, Ammonium-Ion, Wasserstoff-Ion und Gemischen davon, a das Molverhältnis von M1 zu (Al + E) ist und von 0,01 bis 50 variiert, E ein Element ist, ausgewählt aus der Gruppe bestehend aus Gallium, Eisen, Bor, Chrom, Indium und Gemischen davon, x der Molanteil von E ist und von 0 bis 1,0 variiert, n die gewichtete mittlere Valenz von M1 ist und einen Wert von +1 bis +3 aufweist, y' das Molverhältnis von Si zu (Al + E) ist und größer als 3 ist und z" das Molverhältnis von 0 zu (Al + E) ist und einen Wert aufweist, der durch die Gleichung:

$$z'' = (a\cdot n+3+4\cdot y')/2;$$

bestimmt ist, wobei der Zeolith **dadurch gekennzeichnet ist, dass** er ein Röntgendiffraktionsmuster aufweist, das wenigstens die in Tabelle B aufgeführten d-Abstände und relativen Intensitäten aufweist:

Tabelle B

| 2-θ | d (Å) | $I/I_0$ % |
|---|---|---|
| 7,70-8,40 | 11,47-10,52 | m-vs |
| 11,70-12,10 | 7,56-7,31 | w |
| 13,35-14,56 | 6,63-6,08 | s-vs |
| 20,60-21,70 | 4,31-4,09 | w |
| 24,60-25,65 | 3,62-3,47 | m-s |

wobei das Verfahren das Behandeln eines mikroporösen kristallinen Zeoliths gemäß Anspruch 1 zum Austauschen von austauschbaren Kationen gegen andere Kationen oder, im Fall von organischen Kationen, Entfernen durch Erhitzen unter kontrollierten Bedingungen, um einen Ausgangszeolith zu bilden, und Behandeln des Ausgangszeoliths unter Behandlungsbedingungen, um so wenigstens ein Teil des Gerüst-Aluminiums zu entfernen und gegebenenfalls Silicium in das Gerüst einzuführen, umfasst, um den mikroporösen kristallinen Zeolith zu ergeben; wobei der Ausgangszeolith eine empirische Formel auf wasserfreier Basis von:

$$\boldsymbol{M'^{n+}{}_{m'}R^{p+}{}_{r'}Al_{(1-x)}E_{x}Si_{y'}O_{z'}}$$

aufweist, wobei M' ein austauschbares Kation ist, ausgewählt aus der Gruppe bestehend aus Ammonium-Ion, Wasserstoff-Ion, Alkalimetallen, Erdalkalimetallen, Seltenerdmetallen und Gemischen davon, n die gewichtete mittlere Valenz von M' ist und von +1 bis +3 variiert, m' das Molverhältnis von M' zu (Al + E) ist und von 0 bis 5,75 variiert, R Benzyltrimethylammonium (BzTMA) oder eine Kombination von BzTMA und wenigstens einem Organoammonium-Kation, ausgewählt aus der Gruppe bestehend aus protonierten Aminen, protonierten Diaminen, protonierten Alkanolaminen, quaternären Ammoniumionen, diquaternären Ammoniumionen, quaternisierten Alkanolammonium-Ionen und Gemischen davon ist, p die gewichtete mittlere Valenz des organischen Kations ist und von +1 bis +2 variiert, r' das Molverhältnis von R zu (Al + E) ist und von 0 bis 5,75 variiert, r' + m' > 0, y' das Verhältnis von Si zu (Al + E) ist und von größer als 3 bis 25 variiert und z' das Molverhältnis von O zu (Al + E) ist und einen Wert aufweist, der durch die Gleichung:

$$z' = (m' \cdot n + r' \cdot p + 3 + 4 \cdot y')/2,$$

gegeben ist, wobei der Zeolith **dadurch gekennzeichnet ist, dass** er ein Röntgendiffraktionsmuster aufweist, das wenigstens die in Tabelle A aufgeführten d-Abstände und relativen Intensitäten aufweist.

**5.** Verfahren gemäß Anspruch 4, wobei der Behandlungsschritt ausgewählt ist aus der Gruppe bestehend aus Behandlung mit einer/einem Fluorsilicatlösung oder -schlamm, Extraktion mit einer schwachen, starken oder komplexbildenden Säure, Kalzinierung plus Ionenaustausch, Kalzinierung plus Säureextraktion, Dampfbehandlung plus Ionenaustausch, Dampfbehandlung plus Säureextraktion und Gemischen davon.

**6.** Kohlenwasserstoff-Umwandlungsverfahren, umfassend in Kontakt bringen eines Kohlenwasserstoffs mit einem katalytischen Komposit unter Kohlenwasserstoff-Umwandlungsbedingungen, um ein umgewandeltes Produkt zu ergeben, wobei der katalytische Komposit den mikroporösen kristallinen Zeolith gemäß Anspruch 1 umfasst.

**7.** Verfahren gemäß Anspruch 6, wobei das Kohlenwasserstoff-Umwandlungsverfahren ausgewählt ist aus der Gruppe bestehend aus Alkylierung von Aromaten, Isomerisierung von Xylolen, Naphtha-Cracking, Ringöffnung, Transalkylierung, Alkylierung von Isoparaffinen und Isomerisierung von Ethylbenzol.

## Revendications

**1.** Zéolite cristalline microporeuse ayant une composition sous la forme telle que synthétisée et anhydre en termes de rapports molaires des éléments représentée par :

$$M^{n+}{}_m R^{p+}{}_r Al_{(1-x)} E_x Si_y O_z$$

dans laquelle

M est au moins un cation échangeable choisi dans le groupe constitué par les métaux alcalins et alcalinoterreux,
« m » est le rapport molaire de M à (Al+E) et varie de 0 à 0,75,
R est le cation benzyltriméthylammonium (BzTMA) ou une combinaison de BzTMA et d'au moins un cation organoammonium choisi dans le groupe constitué par les cations ammoniums quaternaires, les amines protonées, les diamines protonées, les alcanolamines protonées, les cations di(ammoniums quaternaires), les alcanolamines quaternisées et les mélanges de ceux-ci,
« r » est le rapport molaire de R à (Al+E) et a une valeur de 0,25 à 5,0,
E est un élément choisi dans le groupe constitué par Ga, Fe, In, Cr, B et les mélanges de ceux-ci,
« x » est la fraction molaire de E et varie de 0 à 1,0,
« n » est la valence moyenne pondérée de M et a une valeur de +1 à +2,
« p » est la valence moyenne pondérée de R et a une valeur de +1 à +2,
« y » est le rapport molaire de Si à (Al+E) et varie de plus de 3 à 25 et
« z » est le rapport molaire de O à (Al+E) et a une valeur déterminée par l'équation :

$$z = (m \bullet n + r \bullet p + 3 + 4 \bullet y)/2 \ ;$$

la zéolite étant **caractérisée en ce qu'**elle a un diagramme de diffraction des rayons X ayant au moins les distances réticulaires d et les intensités relatives indiquées dans le tableau A :

Tableau A

| 2-θ | d (Å) | $I/I_0$ (%) |
|---|---|---|
| 3,86-4,22 | 22,87-20,92 | f-m |
| 7,60-7,84 | 11,62-11,27 | F-tF |
| 11,58-11,86 | 7,64-7,46 | f-m |
| 13,29-13,54 | 6,65-6,53 | m |
| 13,90-14,20 | 6,36-6,23 | f |
| 15,34-15,68 | 5,77-5,65 | m |
| 19,30-19,65 | 4,60-4,51 | m |
| 20,37-20,73 | 4,35-4,28 | m-F |
| 23,18-23,54 | 3,83-3,78 | m-F |
| 23,57-23,89 | 3,77-3,72 | F-tF |
| 24,68-25,03 | 3,60-3,55 | m-F |
| 26,84-27,23 | 3,32-3,27 | m |
| 28,15-28,58 | 3,17-3,12 | m |
| 31,25-31,71 | 2,86-2,82 | tF |
| 33,37-33,76 | 2,68-2,65 | f |
| 35,89-36,36 | 2,50-2,47 | m |
| 48,05-48,52 | 1,89-1,87 | f-m |
| 51,38-51,90 | 1,78-1,76 | f-m |
| 55,35-56,04 | 1,66-1,64 | f-m |
| 58,08-58,64 | 1,59-1,57 | f |

**2.** Zéolite selon la revendication 1 dans laquelle « m » vaut zéro.

**3.** Procédé pour la préparation de la zéolite cristalline microporeuse selon la revendication 1 comprenant la formation d'un mélange réactionnel contenant des sources réactives de R, Al, Si et éventuellement E et/ou M et le chauffage du mélange réactionnel à une température de 80 °C à 160 °C, le mélange réactionnel ayant une composition exprimée en termes de rapports molaires des oxydes représentée par :

$$aM_{2/n}O:bR_{2/p}O : (1-c)\ Al_2O_3 :cE_2O_3 : dSiO_2 : eH_2O$$

dans laquelle

« a » a une valeur de 0 à 5,0,
« b » a une valeur de 1 à 120,
« c » a une valeur de 0 à 1,0,
« d » a une valeur de 5 à 100 et
« e » a une valeur de 50 à 15 000.

**4.** Procédé pour la préparation d'une zéolite cristalline microporeuse dans lequel la zéolite cristalline microporeuse a une composition empirique sur une base anhydre en termes de rapports molaires des éléments représentée par :

$$\boldsymbol{M1^{n+}_{a}Al_{(1-x)}\text{-}E_{x}Si_{y'}O_{z''}}$$

dans laquelle

M1 est au moins un cation échangeable choisi dans le groupe constitué par les métaux alcalins, les métaux

alcalinoterreux, les métaux terres rares, l'ion ammonium, l'ion hydrogène et les mélanges de ceux-ci,
a est le rapport molaire de M1 à (Al+E) et varie de 0,01 à 50,
E est un élément choisi dans le groupe constitué par le galium, le fer, le bore, le chrome, l'indium et les mélanges de ceux-ci,
x est la fraction molaire de E et varie de 0 à 1,0,
n est la valence moyenne pondérée de M1 et a une valeur de +1 à +3,
y' est le rapport molaire de Si à (Al+E) et est supérieur à 3 et
z" est le rapport molaire de O à (Al+E) et a une valeur déterminée par l'équation :

$$z'' = (a \bullet n + 3 + 4 \bullet y')/2 \;;$$

la zéolite étant **caractérisée en ce qu'**elle a un diagramme de diffraction des rayons X ayant au moins les distances réticulaires d et les intensités relatives indiquées dans le tableau B :

Tableau B

| 2-θ | d (Å) | $I/I_0$ (%) |
|---|---|---|
| 7,70-8,40 | 11,47-10,52 | m-tF |
| 11,70-12,10 | 7,56-7,31 | f |
| 13,35-14,56 | 6,63-6,08 | F-tF |
| 20,60-21,70 | 4,31-4,09 | f |
| 24,60-25,65 | 3,62-3,47 | m-F |

le procédé comprenant le traitement d'une zéolite cristalline microporeuse telle que définie dans la revendication 1 pour remplacer des cations échangeables par d'autres cations, ou dans le cas de cations organiques leur élimination par chauffage dans des conditions contrôlées pour former une zéolite de départ, et le traitement de la zéolite de départ dans des conditions de traitement ce qui enlève de cette manière au moins une partie de l'aluminium de l'édifice cristallin et insère éventuellement du silicium dans l'édifice cristallin pour fournir la zéolite cristalline microporeuse ; la zéolite de départ ayant une formule empirique sur une base anhydre représentée par :

$$M'^{n+}_{m'}R^{p+}_{r'}Al_{(1-x)}E_xSi_{y'}O_{z'}$$

dans laquelle

M' est un cation échangeable choisi dans le groupe constitué par l'ion ammonium, l'ion hydrogène, les métaux alcalins, les métaux alcalinoterreux, les métaux terres rares et les mélanges de ceux-ci,
n est la valence moyenne pondérée de M' et varie de +1 à +3,
m' est le rapport molaire de M' à (Al+E) et varie de 0 à 5,75,
R est le cation benzyltriméthylammonium (BzTMA) ou une combinaison de BzTMA et d'au moins un cation organoammonium choisi dans le groupe constitué par les amines protonées, les diamines protonées, les alcanolamines protonées, les ions ammoniums quaternaires, les ions di(ammoniums quaternaires), les ions alcanolammoniums quaternisés et les mélanges de ceux-ci,
p est la valence moyenne pondérée du cation organique et varie de +1 à +2,
r' est le rapport molaire de R à (Al+E) et varie de 0 à 5,75,
r' + m' > 0,
y' est le rapport de Si à (Al+E) et varie de plus de 3 à 25 et
z' est le rapport molaire de O à (Al+E) et a une valeur donnée par l'équation :

$$z' = (m' \bullet n + r' \bullet p + 3 + 4 \bullet y')/2,$$

la zéolite étant **caractérisée en ce qu'**elle a un diagramme de diffraction des rayons X ayant au moins les distances réticulaires d et les intensités relatives indiquées dans le tableau A.

5. Procédé selon la revendication 4 dans lequel l'étape de traitement est choisie dans le groupe constitué par le traitement avec une solution ou suspension épaisse de fluorosilicate, l'extraction avec un acide faible, fort ou complexant, la calcination plus l'échange d'ions, la calcination plus l'extraction à l'acide, le traitement à la vapeur d'eau plus l'échange d'ions, le traitement à la vapeur d'eau plus l'extraction à l'acide et les mélanges de ceux-ci.

6. Procédé de conversion d'hydrocarbures comprenant la mise en contact d'un hydrocarbure avec un composite catalytique dans des conditions de conversion d'hydrocarbures pour donner un produit converti, le composite catalytique comprenant la zéolite cristalline microporeuse selon la revendication 1.

7. Procédé selon la revendication 6, le procédé de conversion d'hydrocarbures étant choisi dans le groupe constitué par l'alkylation de composés aromatiques, l'isomérisation de xylènes, le craquage de naphta, l'ouverture de cycle, la transalkylation, l'alkylation d'isoparaffines et l'isomérisation d'éthylbenzène.

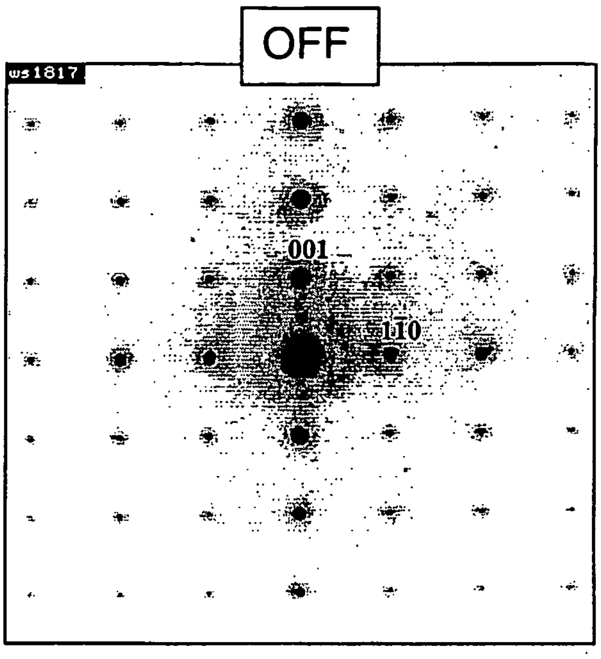


FIG. 1A

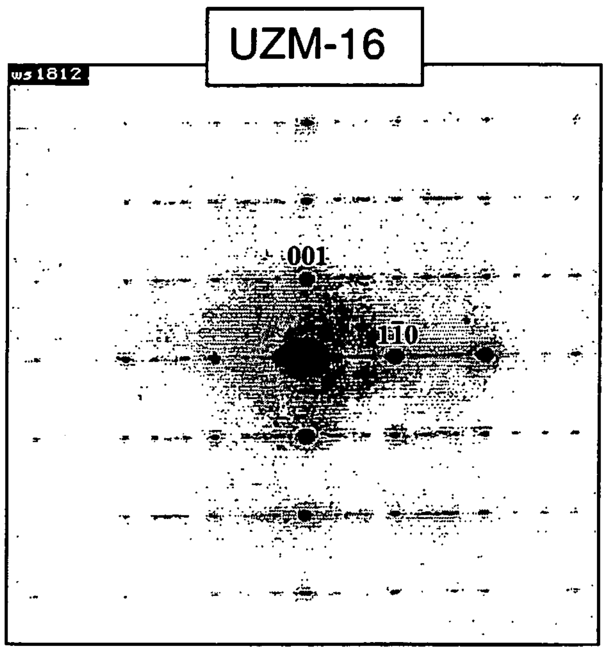


FIG. 1B

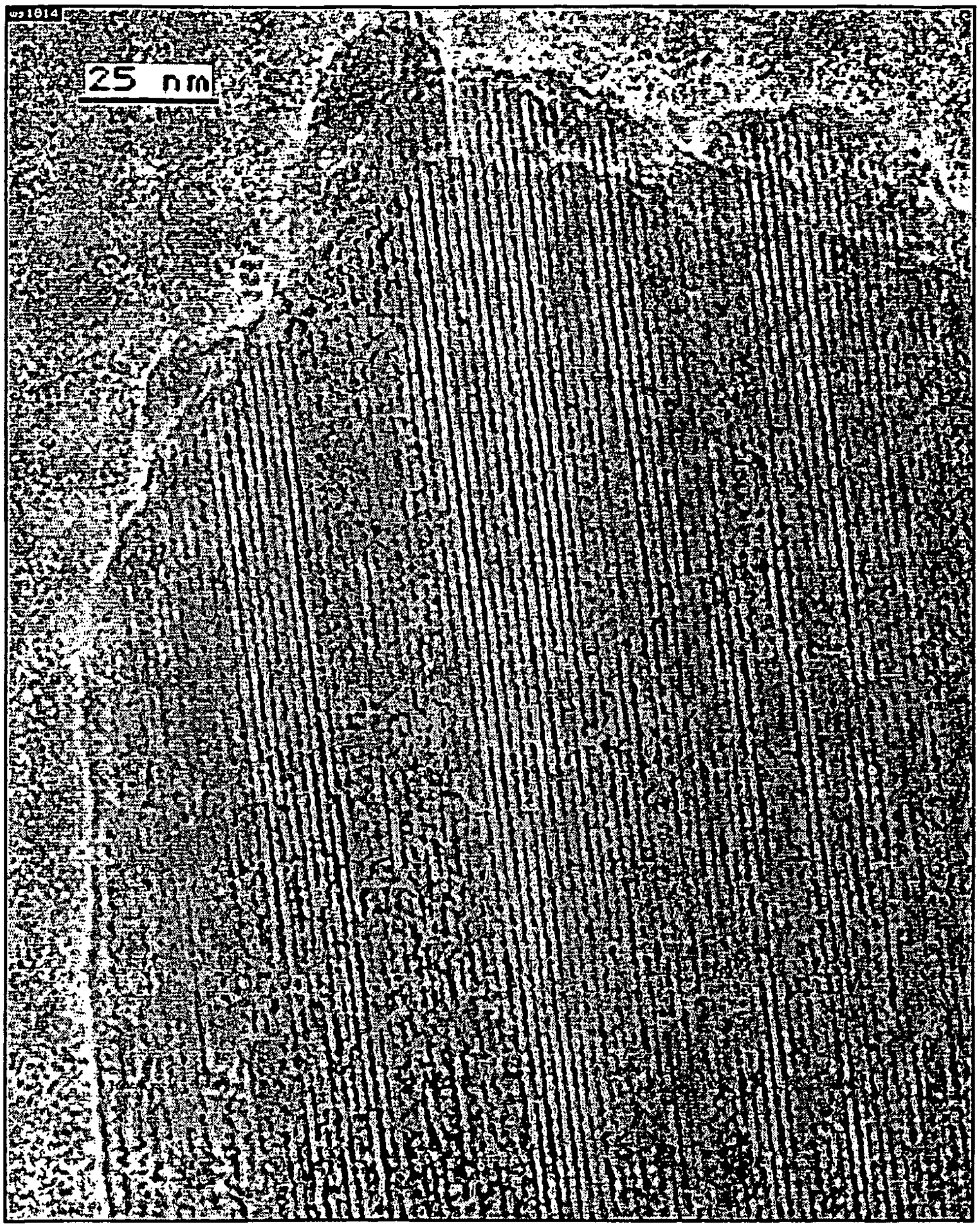


FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2950952 A
- US 3699139 A
- US 3599139 A
- US 4687653 A
- US 6200463 A
- US 4711770 A
- US 4310440 A
- US 4440871 A
- US 5015796 A


### Non-patent literature cited in the description


- **J. M. BENNETT ; J. A. GARD.** *Nature,* 1967, vol. 214, 1005
- **R. AIELLO ; R. BARRER.** *J. Chem. Soc.,* 1970, 1470
- **M. L. OCCELLI ; R. A. INNES ; S. S. POLLACK ; J. V. SANDERS.** *Zeolites,* 1987, vol. 7, 265
- **J. V SANDERS ; M. L. OCCELLI ; R. A. INNES ; S. S. POLLACK.** Studies in Surface Science and Catalysis. Elsevier, 1986, vol. 28, 429
- **D.W. BRECK.** Zeolite Molecular Sieves. John Wiley and Sons, 1974, 636
- **H. PINES.** THE CHEMISTRY OF CATALYTIC HYDROCARBON CONVERSIONS. Academic Press, 1981, 123-154